# EUROPEAN PATENT SPECIFICATION

(11) **EP 1 245 681 B1**
(45) Date of publication and mention of the grant of the patent: **06.06.2007**
(21) Application number: 02005935.8
(22) Date of filing: 15.03.2002
(51) Int. Cl.: C12N 15/55, C12N 9/72

(54) **A method for the production of pharmaceutically active recombinant urokinase comprising the use of butyric acid or its salts**
Verfahren zur Herstellung von pharmazeutisch aktiver, rekombinanter Urokinase umfassend die Verwendung von Buttersäure oder deren Salze
Méthode permettant de produire de l'urokinase recombinante pharmaceutiquement active comprenant l'utilisation de l'acide butyrique ou ses sels

(30) Priority: 16.03.2001 US 815533
(43) Date of publication of application: 02.10.2002
(73) Proprietor: Cerbios-Pharma S.A., CH-6917 Barbengo (CH)
(72) Inventor: Arini, Achille, 6922 Morcote (CH); Coppolecchia, Raffaella, 6922 Morcote (CH); Pagani, Francesca, Paola, 20144 Milan (IT); Herbst, Detlev, 6900 Lugano (CH); Tognini, Antonio, 6605 Locarno (CH)
(74) Representative: Zink-Wild, Markus Peter

(56) References cited:
- EP-A- 0 180 859
- WO-A-00/65070
- DE-A- 2 815 853
- OKABAYASHI K ET AL: "EFFECT OF BUTYRATE ON THE EXPRESSION OF THE HUMAN PREPROUROKINASE GENE INTRODUCED INTO CHINESE HAMSTER OVARY CELLS" CELL STRUCTURE AND FUNCTION, vol. 14, no. 5, 1989, pages 579-586, XP009017114 ISSN: 0386-7196
- CHUPPA S ET AL: "Fermentor temperature as a tool for control for high-density perfusion cultures of mammalian cells" BIOTECHNOLOGY AND BIOENGINEERING. INCLUDING: SYMPOSIUM BIOTECHNOLOGY IN ENERGY PRODUCTION AND CONSERVATION, JOHN WILEY & SONS. NEW YORK, US, vol. 55, no. 2, 1997, pages 328-338, XP002155997 ISSN: 0006-3592
- WHITTAKER J ET AL: "SECRETION OF SOLUBLE FUNCTIONAL INSULIN RECEPTORS BY TRANSFECTED NIH3T3 CELLS" JOURNAL OF BIOLOGICAL CHEMISTRY, vol. 263, no. 7, 1988, pages 3063-3066, XP002254511 ISSN: 0021-9258
- MROCZKOWSKI B ET AL: "EXPRESSION OF HUMAN EPIDERMAL GROWTH FACTOR PRECURSOR COMPLEMENTARY DNA IN TRANSFECTED MOUSE NIH 3T3 CELLS" PROCEEDINGS OF THE NATIONAL ACADEMY OF SCIENCES OF THE UNITED STATES, vol. 85, no. 1, 1988, pages 126-130, XP001155041 1988 ISSN: 0027-8424
- HOVE VAN J L K ET AL: "High-level production of recombinant human lysosomal acid alpha-glucosidase in Chinese hamster ovary cells which targets to heart muscle and corrects glycogen accumulation in fibroblasts from patients with Pompe disease" PROCEEDINGS OF THE NATIONAL ACADEMY OF SCIENCES OF USA, NATIONAL ACADEMY OF SCIENCE. WASHINGTON, US, vol. 93, no. 1, January 1996 (1996-01), pages 65-70, XP002215266 ISSN: 0027-8424

## Description

### Field of the invention

The technical field of the invention is the production of recombinant proteins by means of genetic engineering of eukaryotic cells.

### Background art

Many enzymes or hormones are produced by the cell as inactive pro-enzymes or pro-hormones and are subsequently converted in vivo at the site of "use" or at the "moment of use" into the corresponding active substances. Examples of such enzymes are some matrix metallo proteases, such as collagenase **I**, plasminogen, chymotrypsinogen, trypsinogen. This mechanism of "activation" is also common to hormones such as chromogranin, calcitonin etc.

Among the molecules synthesized by the cell as precursors, Urokinase (urinary type Plasminogen Activator, *u-PA*) can be considered a typical example. This enzyme is a plasma glycoprotein belonging to the wide family of serine proteases. Its main function, among many different ones, is the physiological activation of plasminogen to plasmin (Barlow, G.H, Methods in Enzymology, 45: 239-244, (1976)). Plasmin is a key proteolytic enzyme in the fibrinolytic process that leads to lysis of a clot (Robbins, KC and Summaria, L, Methods in Enzymology , 45: 257-273, (1976)).

In humans *u-PA* is expressed in different tissues as pre-proenzyme (Pre-prourokinase) and is then secreted in the blood as Prourokinase in a single-chain form. This zymogen form is therefore frequently abbreviated with the term *sc-uPA* (*single chain u-PA*). *sc-uPA* has an apparent molecular weight on SDS-PAGE of 50000-54000 Dalton and is catalytically inactive.

Within the blood plasmin performs a proteolytical cleavage which converts the proenzyme *sc-uPA* into a two-chain polypeptide, named *tc-uPA*, which is catalytically and physiologically active.

*tc-uPA* is composed of an A- and a B-chain, linked to each other by a disulfide bond. The B-chain harbors the catalytical site, as well as the N-glycosylation site, located at Asn³⁰² (Bergwerff AA, van Oostrum J, Kamerling JP and Vliegenthart JF, *Eur. J. Biochemistry*,1995, 228: 1009-1019). This form of *uPA* is called *two* *chain High Molecular Weight uPA* (*tc-uPA HMW*). A further proteolytical cleavage operated by plasmin on *tc-uPA HMW* A-chain, leads to the formation of a shorter two chain Urokinase molecule called *tc-uPA LMW* (*two chain u-PA Low Molecular Weight*).

Various attempts in the production of *tc-uPA* by the recombinant DNA technologies confirm on one side the relevance of such a molecule in the clinic and on the other side the need of such an approach required mainly for safety and purity reasons.

EP 154272 describes the production of recombinant glycosylated *sc-uPA* obtained by insertion of the cDNA sequence in animal cells.

EP 303028 describes the production of recombinant glycosylated *sc-uPA* obtained by insertion of the genomic sequence into mammalian cells.

The production described in these patents refers to recombinant Urokinase *(sc-uPA)* in the enzymatically inactive form.

Production of the active enzyme (*tc-uPA*) by recombinant DNA is still an open question, mainly because of its complex extracellular processing. As a matter of fact, low amounts of recombinant active *tc-uPA* is obtained in some of the recombinant eukaryotic systems described so far. In these eukaryotic systems the resulting product is a mixture of *sc-uPA* and *tc-uPA* (Cheng D et al., Chinese Journal of Biotechnology, 1994, 9: 151-159). This is mainly due to the inefficiency of the processing steps involved in *sc-uPA* activation This fact raises some problems for the purification of the two individual forms.

Okabayashi K. et al., Cell Structure and Function, 1989, 14: 579-586, also describe the recombinant production of active enzyme (*tc-uPA*).

The clinical use of a mixture of *HMW* and *LMW uPA* would otherwise involve the problems of a precise reproducibility of the ratio of the relative components, which, moreover, exhibit different pharmacological properties.

On the other side, the production of the recombinant *sc-uPA* led to a different therapeutical strategy, involving the administration of *sc-uPA* as such subsequently converted into the active enzyme within the blood stream, by the endogenous plasmin. In this case the problems arising are due to the uncertainties of the dosage, because of the unpredictability of the conversion rate *in vivo.*

At present, however, the molecular *HMW* and *LMW tc-uPA* forms remain the only molecules of proven therapeutic interest, based on their successful use for many years in the clinical treatment of the clot lysis.

Both molecules, that are currently available as pharmaceutical products, are not recombinant (for a review see: Scripp's thrombolytic report, PJB Publications Ltd., (1993)). The *HMW* form is currently produced by extraction from human urine, as described for example in DE3439980, whereas the *LMW* form has been produced from human foetal kidney cell cultures in the presence of serum as described in DT2551017. In this case serum does not only supply growth factors to the cells, but also plasmin for the conversion of Prourokinase into the *tc-uPA LMW* form. However, safety concerns have been recently raised toward this product, due to its derivation from primary cells ("The pink sheet", February 1, 1999, page 6).

### Summary of the invention

The present invention is related to copy the warding of claim 1.

Further object of the invention is therefore a process for the production, isolation and purification of recombinant *HMW* and *LMW tc-uPA* and the products derived, corresponding to the two forms of mature Urokinase, obtained accordingly to such processes.

### Detailed description of the invention

The present invention is related to a method for the production of mature recombinant two chain-uPA (*tc-uPa*) into the culture medium of genetically modified eukaryotic cells.

The Applicant has now surprisingly found that when genetically modified eukaryotic cells are treated for a time of at least 24 hours with alkanoic acids or their derivatives or salts thereof which are added to the cell culture media, the conversion of a recombinant precursor protein into the corresponding mature form is very efficient and a mature active protein is accumulated at high amount into the cell culture supernatant.

The conversion of a precursor protein into its mature form is particularly efficient when the treatment with alkanoic acids or their derivatives or salts thereof, is combined with the lowering of the cell culture temperature to values equal or below 37°C, preferably comprised between 30°C and 36°C, most preferably 34°C, indicating a synergetic effect.

The term genetically manipulated cells refers to cells which have been transfected or transformed with exogenous DNA, preferably cDNA, encoding for a desired precursor protein. The cDNA sequence is the nucleic acid encoding for the Urokinase precursor (Pre-prourokinase or Prourokinase). The term precursor protein refers to a polypeptide which is secreted or otherwise produced by the cell and which needs further proteolytic cleavage or "processing" for its activation, or for its production in a form as close as possible to the biologically active or natural form. Other examples of such precursor proteins encoded by the corresponding DNA or cDNA sequence are zymogens such as: trypsinogen, chymotrypsinogen, plasminogen, proamylase, prolipase, matrix metallo proteases, i.e. collagenase I, factors belonging to the cascade of the complement system, and prohormones such as: pro-hepatocyte growth factor (pro-HGF), pre-proinsulin, somatostatin, chromogranin-A *etc*.. The precursor protein of the present invention is Pre-prourokinase or Prourokinase, which becomes active in the form of *tc-uPA* (two chain Urokinase). Urokinase (*uPA*) is a serine protease whose main function *in vivo* is the activation of plasminogen to plasmin. In humans *uPA* is expressed in different tissues as pre-proenzyme and then secreted in the blood as Prourokinase, catalytically inactive, in a single chain form (*sc-uPA*), which is in turn processed by plasmin into a two chain *uPA* (*tc-uPA*), catalytically active. The *tc-uPA* is composed by a A- and a B-chain, the latter carrying the catalytic site, linked to each other by a disulphide bond. Active *tc-uPA* is naturally found in the blood and urine in two different forms: the *tc-HMW* and *LMW uPA,* which differ in their A-chain, shorter in *tc-LMW uPA.*

Alkanoic acids, or their derivatives or salts have until now been used in the prior art to enhance the yield of production/secretion of recombinant proteins in mammalian culture systems. According to the present invention, alkanoic acids, or their derivatives or salts thereof act as "processing enhancers". To the best of our knowledge this is the first time that alkanoic acids or their derivatives or salts thereof are used as "processing enhancers" of precursor proteins as defined above.

Alkanoic acids or their salts are chosen among butyrate, preferably sodium butyrate or magnesium butyrate.

Alkanoic acids are added to the culture medium of genetically modified cells in concentration comprised between 0.01 and 500 mM. In the case of mammalian cells the preferred concentration range is comprised between 0.1 and 20 mM, most preferred is 0.5-2.5 mM. It will be appreciated however, that such concentrations may be varied according to the cell line used and according to other factors such as the viability of the cell culture during or at the end of treatment. According to a preferred embodiment, eukaryotic cells are mammalian cells chosen among those commonly used for the production of recombinant proteins: HEK-293 cells, CV-1, COS, BSC-1, MDCK, A-431, BHK, CHO. In a preferred embodiment CHO cells are CHO-Messi cells (ECACC N° 93080520).

A further object of the present invention is a process for the production of recombinant human *tc-uPA* in CHO cells, comprising the addition of butyric acid or salts thereof to a serum-free culture medium where the CHO cell line, genetically modified with an eukaryotic expression vector carrying the Pre-prourokinase cDNA, is maintained for a time between 48 and 200 hours at a temperature comprised between 33 and 35°C, most preferably 34°C.
Further object of the present invention is a process for the conversion of Prourokinase (*sc-uPA*) to tc-urokinase (*tc-uPA*) by addition of butyric acid or salts thereof into the culture media of CHO cells and fermentation of the cell culture at temperatures between 33°C and 35°C and for a period of time between 48 and 200 hours.

As said above for the natural form, also for recombinant Urokinase the term *tc-uPA* refers to the catalytically active Urokinase, which can be in the form of *HMW* and *LMW tc-uPA.* The *HMW* and *LMW tc-uPA* differ for a differentially processed A-chain and show very similar functional activities. The *HMW* and *LMW tc-uPA* may be distinguished e.g. for analytical purposes for a different electrophoretic pattern onto a not-reducing SDS-PAGE: *HMW tc-uPA* migrates at 50-54 KD, while *LMW tc-uPA* migrates at 30-33 KD under the same conditions.

Further object of the present invention is a process for the production of recombinant human *tc-uPA* which is obtained through a process comprising the following steps:
a) culturing genetically manipulated CHO cells transfected with the pre-proUK cDNA or gene in a culture media comprising butyric acid or salts thereof in concentration comprised between 0.1 and 20 mM and at a temperature comprised between 33°C and 35°C, most preferred 34°C;
b) continuing said culture for a period of time of between 48 and 200 hours, most preferably about 120 hours (about five days) and
c) recovering the cell culture supernatant for the isolation of said recombinant human *tc-uPA.*

According to a preferred embodiment of the process of the invention, the genetically manipulated cell line is a stable CHO transformant, most preferably a CHO-Messi (ECACC N. 93080520) transformant selected on the basis of the acquisition of a metabolic marker gene. The culture media is preferably a serum-free culture medium, even more preferably a serum- and protein-free defined medium, such as those commercially available. In a preferred embodiment the cell-culture media is CHOMaster®.

For stable CHO transformant or transfectant is intended a CHO clone transfected with an expression vector which is stably integrated into the cell genome. The eukaryotic expression vector is chosen accordingly to criteria well known in the art: the presence of a strong eukaryotic or viral promoter, such as CMV-IE, SV40 late or early promoter, RSV to drive the transcription of the exogenous DNA; a polyadenylation signal; enhancers of transcription and other regulatory regions which are chosen according to methods well known in the art. Other features of the expression vectors are: a prokaryotic origin of replication, a gene for selection in eukaryotic as well as in prokaryotic cells, such as the β-lactamase gene or kanR, or neoR, or tet-F or Hygromycin-R, as well known by the skilled artisan.

According to a preferred embodiment of the invention, selection in eukaryotic cells is performed by expression of the metabolic marker gene: Trp-Synthase (*trpB* gene) on Trp-auxotrophic CHO cells, or by Histidinol dehydrogenase *(hisD* gene) on His-auxotrophic CHO cells.

Stable clones are preferably selected on the basis of their growth properties, productivity levels and their stability in culture.

Cultivation of the CHO selected clone is usually performed in bioreactor according to protocols well known in the art. According to a preferred embodiment the cultivation is performed in batch. The preferred initial cell concentration is about 3x10⁵ living cells/ml and the cell viability of the inoculum is usually higher than 95%, as measured by the Trypan-Blue exclusion dye method. Usually, the ratio "volume of inoculum/volume fresh medium" varies between 1:1 and 1:5, according also to the total capacity of the bioreactor and to the cell growth.

At a cellular density comprised between 1x10⁶ and 4x10⁶ cells/ml, usually occurring after 3-5 days of growth, cells are separated from the exhausted medium for example by tangential filtration or centrifugation and resuspended in the same original volume of fresh medium, where butyric acid or their salts have been added at final concentrations comprised between 0.1 and 20 mM. Butyric acid or their salts are added at the moment of the cell inoculum or after that. The addition of butyric acid or their salts may be optionally repeated during growth or fermentation. Especially preferred is the sodium or magnesium salt of butyrate which is added at a preferred concentration comprised between 0.5 mM and 2.5 mM, even most preferably comprised between 1 mM and 1.5 mM.

The effect of butyric acid on the production of the recombinant processed protein, is further increased by lowering the temperature of the cell culture to a range of temperatures comprised between 33°C and 35°C, most preferably 34°C± 0.5°C. Glucose levels are also checked during fermentation and preferably maintained above 1g/L. . Batch fermentation during the production phase is performed preferably according to the following parameters:
Temperature: 34°± 1°C, preferentially 34°± 0.5°C
pH: 7.15 ± 0.1
pO₂: 50% ± 20%

According to the preferred fermentation conditions, the higher concentration of *tc-uPA* is obtained after 5 days in culture, after addition of butyric acid, or salts thereof. The production of active product may be followed by functional or immunological assays. *tc-uPA* production is followed for example by a chromogenic assay, such as the Pefachrome® assay. Alternatively the production of active *tc-uPA* or the disappearance of *sc-uPA,* may be followed by SDS-PAGE in denaturing and reducing conditions, because of a different electrophoretic migration pattern: *sc-uPA* migrates in fact as a ~50-54 kD single chain polypeptide, while *tc-uPA HMW* is separated into the A- and B-chain, respectively migrating at ~20 and ~33kD.

According to a preferred embodiment, the exhausted (i.e. the culture medium where cells have been grown) cell culture supernatant containing *tc-uPA* is recovered usually after 3-8 days in culture, usually at the fifth day when the balance between recombinant protein levels and cell viability (the latter kept preferably higher than 70%) is optimal. Alternatively the exhausted supernatant is recovered when *sc-uPA* is absent as measured by SDS reducing PAGE, and maximally converted into *tc-uPA*, where for *tc-uPA* is intended a mixture of the HMW and LMW tc-uPA. Usually the optimal time for the recovery of mature *tc-uPA* is comprised between 48 and 200 hours, with a preferred time of 120 hours of culture in the presence of butyric acid or salts thereof and usually corresponds to a *tc-uPA* production level of about 4000 IU/ml.
According to the described embodiments of the invention, conversion of the precursor forms (pre-pro*uPA*, pro-*uPA*, sc-*uPA*) to the catalytically active *tc-uPA* is characterized by an efficiency higher than 95%, as determined by analytical reducing SDS-PAGE. Of the total *tc-uPA* produced, about 80% is in the *HMW* form and the remaining 20% is in the *LMW* form.

In one of its further embodiments the invention is related to a method for the production of recombinant catalytically active *tc-uPA HMW* and *LMW,* which results from the efficient conversion of the catalytically inactive *sc-uPA* or pro-*uPA* or Prourokinase, directly performed into the exhausted culture medium and characterized by a conversion rate of the precursor into the mature protein higher than 95%.

Recombinant *tc-uPA HMW* and *tc-uPA LMW* molecular forms may be isolated by a chromatographic process characterized by the use of the cell culture supernatant obtained in step c) of the *tc-uPA* production process.

*LMW tc-uPA* may be separated from *HMW tc-uPA* by a process comprising a ion-exchange chromatography and preferably accordingly to the following additional steps: d) acidification of the cell culture supernatant with a weak acid to a pH comprised between 5.0 and 5.8, with the optional addition of a non-ionic detergent and filtration, e) contacting the supernatant with a ion-exchange chromatography column at a pH comprised between 5.5 and 6.5, f) release of the *LMW tc-uPA* by addition of a buffer solution with a pH value comprised between 5.5 and 6.5, further comprising a monovalent ion in concentration comprised between 200 and 300 mM, such as a 250 mM NaCI in phosphate buffer; g) release of the *HMW tc-uPA* by addition of a buffer solution at pH values comprised between 5.5 and 6.5 further comprising monovalent ions in concentration of at least 400 mM, such as a 500 mM NaCI phosphate buffer. Intermediate washings are also performed during chromatography to get rid of all the components not specifically related with *HMW* and *LMW tc-UPA* and are carried out with buffers and/or solutions well known in the art.

Further purification of recombinant *HMW* and *LMW tc-uPA* up to the therapeutical grade is possible, wherein the two forms released respectively in steps g) and f) of the separation process are used and which further comprises an affinity chromatography on benzamidine column. This latter enables the purification procedure to get rid of eventual trace of *sc-uPA* if any.

Benzamidine chromatography is usually performed according to methods well known in the art. *HMW tc-uPA* is purified by benzamidine chromatography further processing the eluate obtained in step g) through the following additional steps: g') contacting the eluate containing *HMW tc-uPA* with a benzamidine column at pH values comprised between 6.2 and 6.8; g") releasing the *tc-uPA HMW* with a buffer solution at pH values comprised between 3.8 and 4.2, further comprising: sodium acetate in concentration ranging between 50 and 150 mM, NaCI in concentration ranging from 300 to 500 mM; g''') further optionally contacting the released *tc-uPA HMW* with a gel-filtration column and releasing the *HMW tc-uPA* with a low-salt phosphate or acetate buffer, such as a 5 mM sodium phosphate buffer, at pH values comprised between 4 and 7.

*LMW tc-uPA* is purified by benzamidine chromatography by further processing the eluate from step f) according to the following additional steps: f') contacting the eluate containing *LMW tc-uPA* with a benzamidine column at pH values comprised between 6 and 8; f'') releasing the *tc-uPA LMW* with a buffer solution at pH values comprised between 3.8 and 4.2 further comprising sodium acetate in concentration comprised between 50 and 150 mM, NaCI in concentration comprised between 300 and 500 mM; f'") further optionally contacting the released *tc-uPA LMW* with a gel-filtration column and releasing the *LMW tc-uPA* with a buffer solution at pH values comprised between 4 and 7, such as a 5 mM sodium phosphate or acetate buffer.

The product(s) obtainable by the combination of the production (steps a through c), separation (steps d through g) and purification processes (steps g' through g"' and f' through f'''), characterized in that the supernatant obtained from the exhausted cell culture medium obtained in step c) of the production process has been used, is recombinant *tc-uPA.* Recombinant *tc-uPA* is in the *HMW* molecular form, as previously defined, and is obtained at a purity level higher than 90%, and/or *tc-uPA* in the *LMW* form, as previously defined, at a purity level higher than 90%, as determined by analytical electrophoresis on SDS-PAGE.

Purified recombinant *tc-uPA* (*HMW* and/or *LMW*) is in the active form, as confirmed by functional and biochemical assays and has a therapeutical grade purity in compliance with the European Pharmacopoeia. Therefore it does not require any further processing and/or purification, as opposed to recombinant pro-*uPA* or *sc-uPA* produced by the recombinant DNA technologies belonging to the prior art. Its molecular form is confirmed by structural data obtained by mass spectroscopy and N-terminal analysis by Edman degradation.

As determined by functional assays such as the clot lysis assay, Michaelis-Menten constant determination, plasminogen activator inhibitor I (PAI-1) binding assay, the recombinant products produced accordingly to the present invention are functionally undistinguishable from the extractive *tc-uPA* as their activities in the assays are fully comparable with those of the extractive, natural product.

Moreover, they are advantageously derived from CHO cells which have a well proven safety in terms of recombinant protein production.

Purified recombinant *tc-uPA HMW* and *LMW* according to the present invention may be used as powerful fibrinolytic agents for the treatment of thrombosis and for any other kind of pathological events, where it is necessary to pharmacologically remove a plasma clot. Their use is supported by the well proven clinical use of the corresponding natural extractive forms.

The recombinant *HMW*/*LMW tc-uPA* obtainable according to the processes of the invention may be used for the treatment of thromboembolytic events requiring the pharmacological removal of clots, such as peripheral arterial occlusion, catheter clearance, pulmonary embolism, deep venous thrombosis or for the treatment of myocardial infarction.

The present invention is described in its best mode of realization by the following experimental examples.

### Description of the drawings

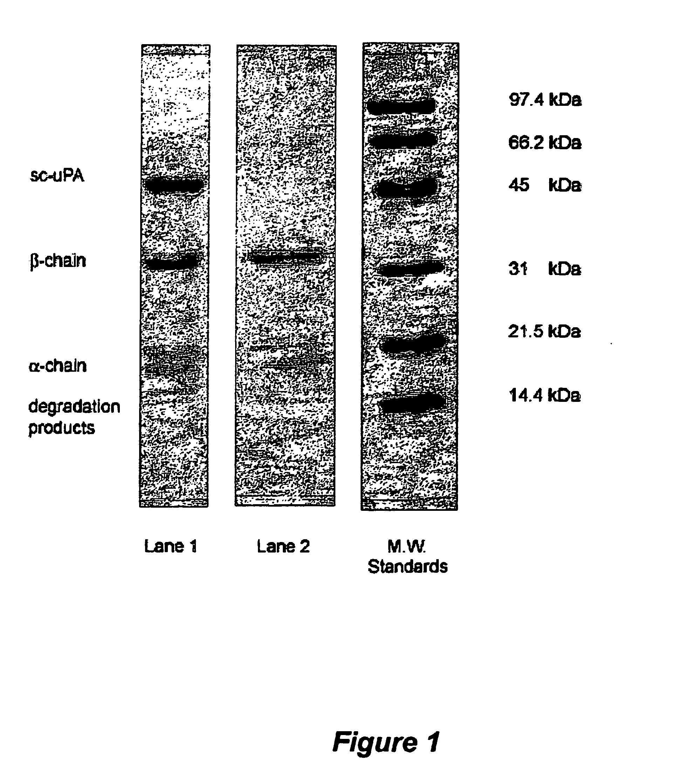

Figure 1: reducing SDS-PAGE of recombinant *tc-uPA.*

The reducing SDS-PAGE of purified *tc-uPA* as obtained after addition of 1.2 mM butyrate to the cell culture medium and 5 days of fermentation is shown.

Under reducing conditions the *tc-uPA* is split in A-chain (20KD) and B-chain (33KD), and the *sc-uPA* runs at about 55KD. Lane 1: purified Urokinase from a recombinant CHO culture grown in the absence of Na-butyrate; lane 2: purified Urokinase from a recombinant CHO culture grown for five days in the presence of 1.2 mM Na-butyrate. Growth conditions are described in the text; lane 3: Molecular weight Standard.

Figure 2: Mass Analysis of recombinant and extractive *tc-uPA*.

The figure shows the spectra obtained with mass spectroscopy analysis of recombinant and extractive *tc-uPA* (*HMW* and *LMW*) in both native (glycosylated; the two upper spectra) and deglycosylated forms (the two lower spectra).

Figure 3: Clot lysis assay.

The experiment was performed by addition of 0.5 ml human Plasma to 100 µl Urokinase (1000 U/ml) and incubated at 42°C for 5 min. 100 µl Thrombin (20 U/ml) were added to the mixture and the absorbance was measured at 660nm during 20 min. at 42°C.

The shaded areas from left to right at "time 0 sec" and at "time 425 sec" do represent: A) recombinant Urokinase; B) extractive Urokinase; C) plasma without addition of thrombin (no clot formation control); D) plasma with thrombin, but without addition of Urokinase (positive clot formation control).

At time 425 sec complete lysis is observed when recombinant or extractive Urokinase are present in the clot.

It is shown that recombinant and extractive Urokinase (*HMW tc-uPA*) exhibit the same clot lysis time.

### EXPERIMENTAL PART

### Example 1: Cloning and selection of stable clones expressing pre-pro-UK.

The cDNA sequence encoding for the human Pre-prourokinase (corresponding to sequence ID D00244 in Genebank) was synthetized from the mRNA of a human kidney cell line (CAKI-1) according to methods well known in the art, described for example in Molecular Cloning: A laboratory Manual, Sambrook et al., Cold Spring Harbor Laboratory press, (1989).

Briefly, cDNA was synthesized by AMV Reverse Transcriptase (Böhringer-Mannheim) reaction in the presence of the mRNA mixture, Oligo dT18 (Böehringer-Mannheim) and a mixture of the four deoxynucleotides (dATP, dGTP, dCTP, dTTP), according to the manufacturer's instructions.

The mixture of cDNA molecules was specifically amplified by PCR with the following 5' and 3' primers:
Oligo1 (5): ^{5'}TAGCGCCGGTACCTCGCCACCATGAGA^{3'}
Oligo2 (3'): ^{5'}TGGAGATGACTCTAGAGCAAAATGACAACCA^{3'}

The 1296 nucleotides long cDNA sequence encoding the human pre-pro-UK sequence was obtained and cloned into a pBR322 derived integrative expression vector carrying the following features:
- the human pre-pro-UK cDNA sequence, under the control of the viral SV40 early promoter (Benoist C. & Chambon P. 1981, Nature 290:304-310),
- the *TrpB* selection marker for the metabolic selection in *CHO-Messi* cells (Hartman, SC Mulligan, RC (1988). Proc. Natl. Acad. Sci. USA, 85: 8047-51),
- Ampicillin resistance (*bla* gene) as an antibiotic resistance marker in *E.coli.*
- the *E. coli* origin of replication for amplification in *E.coli.*

The final recombinant expression vector obtained was called pTZA9.

The CHO cell line used for the production of recombinant *tc-uPA* was the *CHO-Messi* cell line (ECACC, Porton Down, Salisbury, United Kingdom, reg. n° 93080520).

This cell line is able to grow in suspension in chemically defined media CHOMaster® (Ferruccio Messi Cell Culture Technologies, Zürich, Switzerland) without addition of any serum or proteinaceous component. The duplication time of this cell line in such media (complete and selection media) is of about 24 hours.

Transfection of *CHO-Messi* cells with pTZA9 was performed according to the method described in Felgner et al., (1987). Proc. Natl. Acad. Sci. USA, 84: 7413-7417, and respected the recommendations of the transfection's reagent manufacturer. Briefly 1µg DNA expression vector pTZA9 (of a solution of 100µg/ml) was mixed with 30µl Lipofectin® (GIBCO BRL, Life Technologies) in about 500µl exponentially growing *CHO-Messi* cells (about 1x10⁶ cells). The transfection mixture was kept 30min at room temperature, before adding CHOMaster® medium supplemented with 25.1 mg/l Tryptophan and incubating overnight at 37°C.

The *CHO-Messi* cell line is auxotroph for Tryptophan and therefore it absolutely needs this aminoacid for growing. After the transfection of the *CHO-Messi* cells with the Pre-prourokinase expression vector, carrying also the Tryptophan Synthase gene, the *CHO-Messi* cells take advantage of their acquired ability to produce Tryptophan and do manage now to grow in a chemically defined medium in the absence of Tryptophan upon addition of serine and indol.

Stable transfected cells were obtained after limiting dilution in selective CHOMaster® medium (without Tryptophan), with the addition of serine (0.02g/l) and indole (0.35g/l).

Selection of the Urokinase producing clones occurred by dilution of the transfection mixture with selective CHOMaster® medium in microtiter wells. Another system used for obtaining selected single clones producing Urokinase was to dilute 1:10 a cell suspension of about 10³ cells/ml with a highly viscous solution of 0.2 g/l Methocel in selective CHOMaster® medium with addition of 4% dialysed foetal calf serum. The viscous mixture was seeded onto a 24-well cluster plate and after 2 weeks single clones were picked up with a sterile pipette tip and a new culture in suspension was started.

The following culture strategy was adopted for the preparation of the inoculum in the bioreactor. A cell culture raised in microtiter wells was further split 1:3 with selective CHOMaster® medium in larger wells (24-well cluster and 6-well cluster plates) and subsequently in 25- and 75 cm²-T flasks once the cell density reached about 4-5 x 10⁵ cells/ml.

Keeping constant the splitting ratio (1:3) and the cell density at which the split in the T-flasks occurred, the cultures in suspension were transferred into 2L spinner flasks (Integra Bioscience, Switzerland) and a working volume of 1L was used during this growth process.

### Example 2: Determination of the optimal conditions for tc-uPA secretion/processing.

At first, the optimal conditions related to butyrate concentration and temperature in a laboratory scale were determined. Preliminary experiments aimed at optimizing the Na-butyrate concentration were performed in a 1 L culture of the recombinant CHO cell line (1x10⁶ cells/ml), in order to achieve the best *tc-uPA* (*HMW* and *LMW*) production and the higher cell viability after 5 days of cultivation in spinner-flasks.

In table 1 are reported the cell viability and the *uPA* yield after 5 days cultivation in 1L Spinner-flask culture at 37°C and at different Na-butyrate concentrations. *uPA* activity was followed by a chromogenic assay using the Pefachrome® UK (54-46) (Pentapharm, LTD, Basel, Switzerland) as chromogenic substrate, according to the manufacturer's instructions; the cell viability was followed every day and measured microscopically after Trypan blue dye-exclusion method, as described in Doyle et al. A, Griffiths, JB and Newell, DG (Eds.) (1994), in "Cell & Tissue Culture: Laboratory Procedures". John Wiley & Sons . New York.

**Table 1**

| Na-Butyrate in 1L Spinner-flask culture | Activity of *tc-uPA* (IU/ml) | Cell viability |
|---|---|---|
| 0 (control) | 320 | 92% |
| 0.6mM | 526 | 83% |
| 1.2mM | 959 | 76% |
| 2.5mM | 361 | 56% |

As shown in Table 1, the most effective Na-Butyrate concentration in terms of *uPA* yield is 1.2mM after 5 days of cultivation in a 1L Spinner-flasks at 37°C. At this concentration the cell viability is still reasonably high (76%), even if compared with the control (92%). A high cellular viability prevents a too extensive contamination of the supernatant with cellular debris and host related proteins and reduces the load of contaminants to be eliminated during the purification process.

A second set of experiments was performed at 1.2 mM butyrate, in a 5-days fermentation to determine the optimal temperature in a 2L culture (1.5-2x10⁶cells/ml) of the recombinant CHO cell line in bioreactor.

**Table 2**

| Temperature (1.2 mM Na-Butyrate) | Activity of *tc-uPA* (IU/ml) (5 days cultivation) | Cell viability |
|---|---|---|
| 37°C (control) | 1830 | 53% |
| 34°C | 5043 | 67% |
| 32°C | 800 | 84% |

In table 2 it is shown that 34°C is the temperature which allows a high *uPA* productivity rate together with high cell viability. Interestingly, when the same experiment was performed at 32°C, an activity of 3961 U/ml was reached only after 12 days with fower cell viability (63%).

The reduction of the cultivation time is highly desirable for economic reasons as well as to maintain the integrity of the protein. It is in fact desirable to avoid a long exposure of the recombinant product to different proteolytic and glycolytic enzymes derived from lysed cells.

From the data shown in Table 2 it appears that lowering the cultivation temperature to 34°C, together with the addition of Na-Butyrate (1.2mM) to the culture medium, determines a synergetic effect resulting in a better yield of active *uPA.*

From these data it can be deduced that the 1.2 mM Na-Butyrate treatment at 34°C temperature are optimal conditions for active *tc-uPA* production and cell viability, in particular for a 5 days cultivation batch.

### Example 3: Production of recombinant tc-uPA in CHO cells in a 2L bioreactor.

The cell inoculum was performed in a 2.4 L bioreactor (Infors HT, Type Labforce, Bottmingen, Switzerland) by splitting an exponentially growing cell culture. The ratio "volume of inoculum/volume fresh medium" was chosen between 1:1 and 1:5, according to the total capacity of the bioreactor and to the culture conditions. A working volume of 2L and a final cell density of the inoculum of 3x10⁵ cells/ml were chosen.

The culture used for the primary inoculum had a viability ratio not lower than 95%. During the growth in the bioreactor the culture in suspension was monitored for the concentration of glucose, which was never kept lower than 1 g/L in the exhausted medium

The following fermentation parameters were set up for the culture:

| | |
|---|---|
| Temperature: | 37°± 0.5°C, |
| pH: | 7.15 ± 0.1 |
| pO₂: | 50% ± 20% |

When the cellular density reached values of about 2 x 10⁶ living cells/ml, the cells were separated from the exhausted medium by tangential filtration (or centrifugation). Cells were then resuspended in the bioreactor in the same original volume of fresh medium CHOMaster® with the addition of sodium butyrate to achieve a final concentration of 1.2 mM.

The temperature of the culture was lowered to 34° ± 0.5°C, and the other fermentation parameters were kept as previously set, as follows:

| | |
|---|---|
| Temperature: | 34°C ± 0.5°C, |
| pH: | 7.15 ± 0.1 |
| pO₂: | 50% ± 20% |

The production of active u-PA (*tc-uPA HMW* and *LMW*) was monitored by a chromogenic test on a specific substrate, Pefachrome® UK. A progressive increase of the activity was observed up to maximal values as high as 7000 IU/ml, achieved after 4-5 days of fermentation. At this point the cells were harvested and subsequently discarded and the exhausted culture medium, containing *tc-uPA*, was further processed for purification.

The average production levels relative to four independent fermentations are presented in table 3:

**Table 3. Fermentation data**

| Days after the addition of 1.2 mM Na-butyrate | Activity on the chromogenic substrate (IU/ml) | Cell viability |
|---|---|---|
| 1 | 155 ± 23% | 87 ± 6% |
| 2 | 662 ± 32% | 81 ± 12% |
| 3 | 1995 ± 13% | 78 ± 15% |
| 4 | 3170 ± 23% | 72 ± 14% |
| 5 | 5043 ± 13% | 67 ± 10% |

In the bioreactor a gradual decrease of the cell viability was observed from the first to the fifth day in culture after the addition of sodium butyrate. The minimal cell viability value at 1.2 mM butyrate was observed at the fifth day in culture and was not significantly lower than 70% and is therefore still relatively high. At these values a limited release of lysosomal degradation enzymes into the growth medium is expected.

As shown in table 3, the dramatic increase in the expression of *tc-uPA* during the production process as it is described, occurs mainly during the last 2/3 days of fermentation after the addition of sodium butyrate. The permanence of most of the secreted Urokinase into the exhausted medium, is optimal when limited to 2/3 days, at a temperature of 34°C. This combination of parameters reduces the exposure of the protein to the degradation activity of the proteolytic and glycolytic enzymes and allows for good quality of the recombinant protein to be purified. In figure 1 are shown the products obtained after fermentation in the absence or in the presence of butyrate 1.2 mM, at a temperature of 34°C. In the latter conditions the complete conversion of *sc-uPA* into *tc-uPA* can be appreciated, when compared to a culture where no Na-butyrate was added.

### Example 4: Purification of tc-uPA HMW and LMW

### Purification of tc-uPA HMW.

The supernatant of the cell culture grown in bioreactor, obtained as described in Example 3 was acidified by addition of CH₃COOH to a pH of 5.5 and cleared from cellular debris by filtration on 0.45µm filter. Tween-80 0.01% was added and the supernatant was loaded onto an ion exchange chromatography column (SP Sepharose Big Beads, Amersham-Pharmacia) previously equilibrated with a 20 mM pH 6.0 sodium phosphate buffer solution. The column bed size was 10 cm height, 2.6 cm diameter. The flow rate during loading and wash was 10 ml/min and during elution was 2 ml/min.

After loading, the column was first washed with 3 volumes of 20 mM sodium phosphate, 150 mM NaCI, pH 6 buffer solution, in order to remove the non-Urokinase related impurities and subsequently with 3 more volumes of 20 mM sodium phosphate, 250 mM NaCI, pH 6.0 buffer solution, in order to mainly elute *tc-uPA LMW.* This latter is stored frozen for a further purification.

The elution of *tc-uPA HMW was* performed by passing through the ion exchange column, a 20 mM sodium phosphate, 500 mM sodium chloride, pH 6.0 buffer solution.

The eluate obtained, containing Urokinase (*tc-uPA HMW*) was brought to pH 6.5 by the addition of 1N NaOH. Then it was loaded onto a benzamidine Sepharose 6B affinity column, previously equilibrated with at least 2 volumes of a 20 mM sodium phosphate, 400 mM NaCI, pH 6.5 buffer solution.

The bed size of the column was 10 cm height, 2.6 cm diameter. The flow rate during the load, wash and elution steps was 2.5 ml/min.

The column was subsequently washed with 2 volumes of a 20 mM sodium phosphate, 400 mM NaCI, pH 6.5 buffer solution and finally eluted with 2.5 volumes of 100 mM sodium acetate, 400 mM NaCI, pH 4.0 buffer solution.

The benzamidine column allowed to get rid of the non-Urokinase impurities, as well as of the undetectable amount, if any, of sc-*uPA* that could be present in the load.

The *tc-uPA HMW* containing fractions were identified as belonging to a relatively consistent and unique peak in the chromatogram and were pooled together.

The obtained pool was loaded onto a gel filtration column (separation on the basis of molecular size exclusion). The gel filtration column had 30 cm height and 2.6 cm diameter, and was previously equilibrated with a 5 mM sodium phosphate pH 4.9 buffer solution. The applied flow rate was about 3 ml/min.

Urokinase *tc-uPA HMW* was thus eluted in its pure form, by running the elution with a 5 mM sodium phosphate pH 4.9 buffer solution, as shown in figure 1 (lane2).

Urokinase was finally ready to be formulated in a buffer suitable for a terminal lyophilisation.

### Purification of LMW tc-uPA

The fraction(s) corresponding to the *tc-uPA LMW,* collected from the wash of the ion exchange column with 20 mM sodium phosphate, 250 mM NaCl, pH 6.0 buffer solution, were pooled and subsequently purified through an affinity benzamidine chromatography column. Before being loaded onto this column, the pool was adjusted to pH 6.5 or 7.0 by addition of 1N NaOH and the column was previously equilibrated with at least 2 volumes of a 20 mM sodium phosphate, 400 mM NaCI, pH 6.5 buffer solution. After the load, the column was washed with 2 volumes of 20 mM sodium phosphate, 400 mM NaCI, pH 6.5 buffer solution and finally eluted with 2.5 volumes of 100 mM sodium acetate, 400 mM NaCI, pH 4.0 buffer solution. The *tc-uPA LMW* containing fractions were identified as belonging to a relatively consistent and unique peak in the chromatogram and were pooled together.

The obtained pool is loaded onto a gel filtration column (separation on the basis of molecular size exclusion) of 30 cm height and 2.6 cm diameter, previously equilibrated with a 5 mM sodium phosphate pH 4.9 buffer solution. The applied flow rate is about 3 ml/min.

Urokinase *tc-uPA LMW is* thus eluted in its pure form, by running the elution with a 5 mM, sodium phosphate pH 4.9 buffer solution and it is finally ready to be formulated in a buffer suitable for a terminal lyophilisation.

### Example 5: Characterization of recombinant tc-uPA.

### Processing of the recombinant molecule

Characterization of recombinant *tc-uPA HMW* was carried out in comparative studies with the commercial extractive *tc-uPA HMW* (Ukidan®, Serono) by mass spectroscopy and functional studies.

### Molecular mass

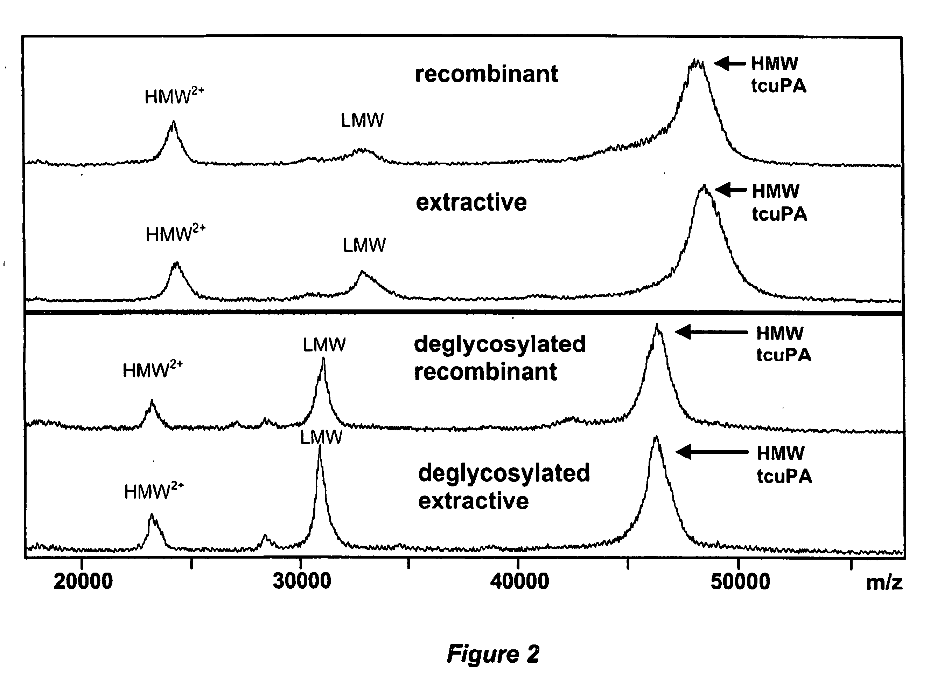

The mass spectroscopy data, the accuracy of which may vary in the range of +/-50 Da to +/-100 Da, confirmed that the molecular mass corresponded to what expected after correct processing of the precursor (*sc-uPA*) protein and in particular, as shown in figure 2, that:
- the recombinant, produced accordingly to the process of the invention, and the commercial extractive *HMW tc-uPA* had very similar molecular masses of 48267 Da and 48565 Da, respectively;
- when deglycosylated, the two forms had also very similar molecular masses of 46382 (recombinant) and 46313 Da (extractive);
- similarly, the analysis of the recombinant produced accordingly to the process of the invention and extractive *LMW tc-uPA* exhibited very close masses in the glycosylated (33249 Da and 33189 Da, respectively) and not glycosylated form (31029 and 30969, respectively).

Moreover, MALDI-MS mass spectroscopy analysis showed that the purified molecules are largely intact (>95%), in other words it confirmed that the *uPA*-related degradation products are present in non-relevant amount and that therefore the chosen purification procedures do not affect the integrity of the recombinant molecule.

The correct processing at the N-terminal was confirmed through the Edman degradation of purified *HMW tc-uPA.* As expected, the NH₂-terminal sequence of the B-chain was determined as:
**-IIGGEF-,**
whereas the NH₂-terminal sequence of the A-chain was, as expected:
**-SNELHQ-,**

These data demonstrated that the proteolytic cleavage occurs exactly and specifically at the Lys¹⁵⁸-Ile¹⁵⁹ bond, and Lys¹⁵⁸ is correctly removed from the rest of the molecule. Moreover, the analysis of the peptide mapping confirmed the existence of correct NH₂- and C-termini of both A- and B-chains of the recombinant *tc-uPA HMW.*

### Glycosylation pattern

Glycans were analyzed on purified recombinant *tc-uPA* by mass spectroscopy and fluorescence-assisted carbohydrate electrophoresis (FACE). Both methods revealed stable glycosylation: recombinant Urokinase-derived N-glycans consist of core-fucosylated, two-, three- and four antennary complex chains with a sialylation degree of 80-90%. The glycosylation site was determined by mass spectroscopy, and confirmed that the glycosylation at Asn³⁰² on *tc-uPA* has occurred.

### Functional studies

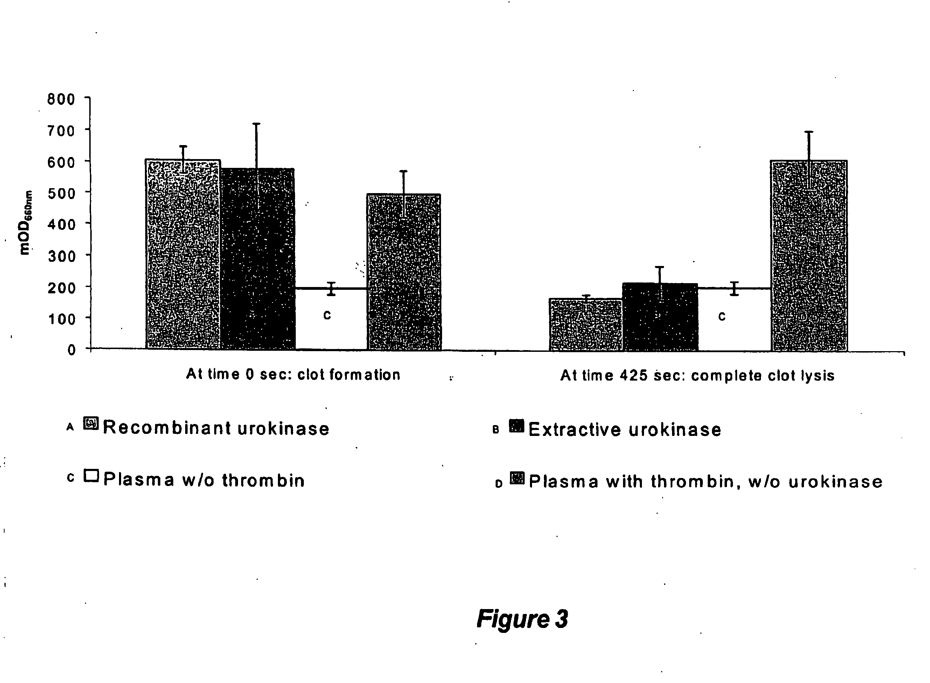

The biological activity of recombinant *HMW tc-uPA* was also determined by measuring:
- analysis of K_{d} of binding to natural Urokinase receptor (see Table 4);
- analysis of the stoichiometric inhibitory activity of Plasminogen activation inhibitor (PAI-1), which is the natural inhibitor of Urokinase;
- analysis of the kinetic of inhibition by PAI-1 (see Table 4);
- study of enzymatic parameter Kₘ on chromogenic substrate (see Table 4);
- study of plasminogen activation kinetics;
- clot lysis capacity (Figure 3);
- fibrin degradation.

**Table 4**

| | | | |
|---|---|---|---|
| *Sample* | *k₁ (M⁻¹*s⁻¹)*10⁻⁷* | *Kₘ (mM)* | *k_{d} (nM)* |
| recombinant *HMW tc-uPA* | 2.07 ± 0.57 | 0.027 ± 0.0018 | 1.24 |
| Extractive *HMW tc-uPA* | 2.59 ± 0.20 | 0.028 ± 0.0050 | 1.77 |

The data shown in table 4 demonstrate the substantial functional identity of the recombinant *HMW tc-uPA*, produced accordingly to the process of the invention, and the commercial extractive *HMW tc-uPA*. In particular it is shown that the two products had:
(a) similar rate constants (*k₁*) for the complex formation with PAI-1, as measured according to Chmielweska et al. Biochem. J. 1988, 251:327-332,
(b) similar Michaelis-Menten constants (*Kₘ*) for the Urokinase substrate, as measured according to Briggs, GE and Haldane JBS, Biochem. J. 1925, 29:338-339 and Lijnen, HR et al. Eur. J. Biochem. 1994, 224:567-574, and
(c) similar affinity constants for the Urokinase receptor (*K_{d}*), as measured according to: Cubellis, M.V. et al., J. Biol. Chem., 1986, 261:15819-15822.

## Claims

1. A method for the production of a mature recombinant two chain-*uPA* (*tc-uPA*) into the culture medium of an eukaryotic cell line genetically transfected with a cloned precursor cDNA sequence, comprising an incubation at a temperature between 33°C and 35°C of said cell line in the cell culture medium wherein butyric acid or its salts have been added for a time between 48 and 200 hours, and whereby the efficiency of the conversion of the precursor, i.e. single chain-*uPA* (*sc-uPA*) into the active two chain-uPA (*tc-uPA*) is higher than 95 %.

2. A method according to claim 1 wherein said cDNA sequence encodes for a protein precursor.

3. A method according to claim 2 wherein said precursor cDNA sequence encodes for the human Pre-prourokinase.

4. A method according to claim 1 wherein the two chain *uPA* is *HMW.*

5. A method according to claim 1 wherein the two chain *uPA* is *LMW.*

6. A method according to claim 1 wherein said butyric acid salts are chosen among sodium butyrate and magnesium butyrate.

7. A method according to claim 1 wherein said eukaryotic cell line is a mammalian cell line chosen among: HEK-293, CV-1, COS, BSC-1, MDCK, A-431, CHO, BHK, CHO-Messi.

8. A method according to claim 1 wherein said cell culture medium is serum-free.

## Patentansprüche

1. Verfahren zur Herstellung einer vollentwickelten rekombinanten zweikettigen-*uPA (tc-uPA)* im Kulturmedium einer eukaryotischen Zelllinie, die mit einer geklonten Vorläufer cDNA Sequenz transfektiert ist, umfassend eine Inkubation bei einer Temperatur zwischen 33°C und 35°C der genannten Zelllinie im Zellkulturmedium, worin Buttersäure oder ihre Salze hinzugegeben worden sind, während einer Zeit zwischen 48 und 200 Stunden, und wobei die Wirksamkeit der Umwandlung des Vorläufers, d.h. einkettige-*uPA* (*sc-uPA*) in die aktive zweikettige-*uPA (tc-uPA)* höher als 95 % ist.

2. Verfahren nach Anspruch 1, **dadurch gekennzeichnet, dass** die genannte cDNA Sequenz einen Proteinvorläufer kodiert.

3. Verfahren nach Anspruch 2, **dadurch gekennzeichnet, dass** die genannte Vorläufer cDNA Sequenz menschliche Pre-prourokinase kodiert.

4. Verfahren nach Anspruch 1, **dadurch gekennzeichnet, dass** die zweikettige *uPA HMW* ist.

5. Verfahren nach Anspruch 1, **dadurch gekennzeichnet, dass** die zweikettige *uPA LMW* ist.

6. Verfahren nach Anspruch 1, **dadurch gekennzeichnet, dass** die genannten Buttersäuresalze ausgewählt sind aus Natriumbutyrat und Magnesiumbutyrat.

7. Verfahren nach Anspruch 1, **dadurch gekennzeichnet, dass** die genannte eukaryotische Zelllinie eine Zelllinie eines Säugers ist, ausgewählt aus HEK-293, CV-1, COS, BSC-1, MDCK, A-431, CHO, BHK, CHO-Messi.

8. Verfahren nach Anspruch 1, **dadurch gekennzeichnet, dass** das genannte Zellkulturmedium serumfrei ist.

## Revendications

1. Méthode de production d'une *uPA* bicaténaire recombinante mature (*tc-uPA*) dans le milieu de culture d'une lignée de cellules eucaryotes génétiquement transfectées avec une séquence d'ADN complémentaire précurseur clonée, comprenant une incubation à une température comprise entre 33 et 35 °C de ladite lignée de cellules dans le milieu de culture cellulaire, dans lequel de l'acide butyrique ou ses sels ont été ajoutés pendant une période comprise entre 48 et 200 heures, et dans laquelle méthode, le rendement de la conversion du précurseur, c'est-à-dire de l'*uPA* monocaténaire (*sc-uPA*) dans l'*uPA* bicaténaire active (*tc-uPA*) est supérieur à 95 %.

2. Méthode selon la revendication 1, dans laquelle ladite séquence d'ADN complémentaire code pour un précurseur de protéine.

3. Méthode selon la revendication 2, dans laquelle ladite séquence d'ADN complémentaire précurseur code pour la pré-prourokinase humaine.

4. Méthode selon la revendication 1, dans laquelle *l'uPA* bicaténaire est HMW.

5. Méthode selon la revendication 1, dans laquelle l'*uPA* bicaténaire est LMW.

6. Méthode selon la revendication 1, dans laquelle lesdits sels de l'acide butyrique sont sélectionnés parmi le butyrate de sodium et le butyrate de magnésium.

7. Méthode selon la revendication 1, dans laquelle ladite lignée de cellules eucaryotes est une lignée de cellules de mammifères, sélectionnée parmi: HEK-293, CV-1, COS, BSC-1, MDCK, A-431, CHO, BHK et CHO-Messi.

8. Méthode selon la revendication 1, dans laquelle ledit milieu de culture cellulaire est exempt de sérum.
